Europäisches Patentamt

European Patent Office

Office européen des brevets

(11) Numéro de publication : **0 677 277 A2**

# (12) DEMANDE DE BREVET EUROPEEN

(21) Numéro de dépôt : **95400558.3**

(22) Date de dépôt : **15.03.95**

(51) Int. Cl.⁶ : **A61B 17/70, A61B 17/88**

(30) Priorité : **18.03.94 FR 9403189**
**14.12.94 FR 9415049**
**10.02.95 FR 9501561**

(43) Date de publication de la demande :
**18.10.95 Bulletin 95/42**

(84) Etats contractants désignés :
**AT BE CH DE DK ES FR GB GR IE IT LI LU MC NL PT SE**

(71) Demandeur : **Moreau, Patrice**
**6 Avenue du Parc de Sceaux**
**F-92160 Antony (FR)**

(71) Demandeur : **Elberg, Jean François**
**7 Rue Héliopolis**
**F-75017 Paris (FR)**

(72) Inventeur : **Moreau, Patrice**
**6 Avenue du Parc de Sceaux**
**F-92160 Antony (FR)**
Inventeur : **Elberg, Jean François**
**7 Rue Héliopolis**
**F-75017 Paris (FR)**

(54) **Ensemble prothétique rachidien.**

(57) Ensemble prothétique rachidien comportant en nombre convenable, des éléments prothétiques de liaison intervertébraux élastiques avec amorrissement présentant au moins une conformation dont l'une dite omnidirectionnelle (2a) comprend deux vis pédiculaires assujetties ; l'une sur la vertébre dite haute (4a), l'autre sur la vertébre dite basse (4b) et recevant des moyens d'ancrage (5-6) réunis par un dispositif omnidirectionnel à queues (9) avec demi-colliers à empreintes circulaires (5a-5b) et (6a-6b) enserrant et fixant les queues cylindriques (9a-9b).

FIG-7

EP 0 677 277 A2

L'invention concerne les prothèses pouvant être appliquées sur la totalité des vertèbres du rachis.

Un rachis normal peut être considéré en bonne approximation à la fois comme un système élastique ou chaque liaison intervertébrale reprend sa position d'origine dès que l'effort appliqué a cessé et qui est associé à un système avec amortissement dans la mesure ou l'ènergie résultant des chocs divers est dissipée sous forme de chaleur et n'est pas restitué au système élastique sous forme mécanique.

Les contraintes appliquées sur un rachis normal peuvent être trés importantes et l'on estime par exemple, que la vertébre L3 supportant une charge normale de 70 daN, peut subir une surcharge de 120 daN pour un sujet penché à 20° et 340 daN en soulevant seulement un poids de 20 Kg jambes tendues.

On sait d'autre part que ce même rachis peut enregistrer jusqu'à 2 millions en moyenne de sollicitations et chocs de diffèrentes natures par années soit une centaine de millions de cycles de contraintes durant la vie active moyenne d'un individu. Toutes les prothèses rachidiennes doivent donc répondre idéalement à plusieurs objectifs aussi proches que possible des conditions rencontrées "in vivo" en particulier: d'élasticité, d'amortissement et de tenue en fatigue.

Ces domaines ont été trés recherchés et l'on trouve une abondante littérature sur ce sujet ainsi que divers brevets.

Parmi la littérature on peut citer:Three-dimensional biomechanical properties of the human cervical spine in vitro(European spine journal 1993),
- A biomechanical analysis of short segment spinal fixation using a tree-domensional geometric and mechanical model (spine vol.18 number 5. P.P 536-545 -1993),
- Three dimensional geometrical and mechanical modeling of the lumbar spine (biomechanical vol. 25 N° 10 P.P 1164/1992),
  Influence of geometrical factors on the behavior of lumbar spine segments: a finite element analysis (Europeean spine journal -1994),
- comportement bio-mécanique d'un ressort inter-apophysaire vertébral postérieur. Analyse expérimentale du comportement discal en compression. (rachis 1993- Vol 5 N°2)

Parmi les brevets, on peut citer:
- le N°F 2681525 qui concerne un amortisseur seul interépineux,
- le N°F 2683445 qui présente un autre type d'amortisseur seul à plaques.
- le N°EP 516567 qui dispose un amortisseur seul avec tête rotulante,
- le N°EP 576379 qui décrit un amortisseur seul avec limites de débattements axiaux,
- le N° EP 538183 qui montre une pastille intervertébrale souple mise en place par griffes en lieu et place du nucléus pulposus.

Toutes ces études et brevets ne visent que des fonctions dissociées soit d'amortissement soit d'élasticité et n'entrent pas de ce fait dans le champ d'application de l'invention.

De plus, l'invention vise un procédé permettant de paramètrer les éléments spécifiques d'une prothèse intervertébrale consistant à déterminer par tous moyens appropriés tels que radiographie,scanner,imagerie RM, les défauts à corriger en rapport avec l'anomalie constatée, à analyser ces défauts, à modéliser la prothèse, à la soumettre après modélisation à des essais de fatigue sur un banc de fatigue spécialisé, puis aprés caractérisation,à procéder à son implantation sur le patient.

l'invention permet en outre, par un dispositif de liaison intervertébrale approprié, associé à des moyens d'ancrage sur les vertébres, d'orienter l'action du moyen élastique et d'amortissement dudit dispositif de liaison de telle sorte que le praticien puisse corriger certaines affections telles que scolioses par exemple.

D'une manière générale, toutes les liaisons interpédiculaires consistent pour le praticien:
- à fixer les vis pédiculaires sur les deux vertébres adjacentes intéréssées,
- à utiliser la partie dépassante de ces vis pour assujettir des moyens d'ancrage avec leur système de blocage adapté,
- à relier au moins deux moyens d'ancrage adjacents par un dispositif de liaison omnidirectionnel ou directionnel qui aprés blocage, réalise l'immobilisation recherchée,
  sans perdre de vue le fait que le geste opératoire doit être aidé par la facilité de pose des divers constituants.

L'invention, exposée ci-aprés à l'aide des dessins annexés,comprend deux parties afin d'en assurer une meilleure compréhension:
. d'une part, l'explication théorique mettant en évidence l'apparition de la nécessité de mettre en oeuvre une prothèse intervertébrale,
. d'autre part, la description du fonctionnement de la prothèse selon l'invention s'insèrant dans un ensemble rachidien et comportant au moins un élément intervertébral incluant sur deux vis pédiculaires, deux moyens d'ancrage recevant un dispositif de liaison élastique avec amortissement qui peut être omnidirectionnel ou directionnel et avoir été modélisé avant implantation.

Sur les dessins:
- la figure 1 est une représentation schèmatique montrant à titre d'exemple, le positionnement de prothèses selon l'invention sur un segment rachidien entre les vertèbres L5 à L1 et D12 et vu en coupe partielle unilatèrale,
- la figure 2 est une vue trés schèmatique montrant le comportement mécanique possible des deux vertèbres L5-L4 les plus chargées et prises comme référence,
- les figures 3 et 4 sont des graphiques montrant respectivement les contraintes en fatigue pouvant être appliquées sur le rachis et le mode de travail type d'un ressort;
  agissant conjointement ou non avec un amortisseur,
- les figures 5 et 6 représentent en perspective, d'une part,un dispositif de liaison directionnel avec représenté symboliquement un banc d'essais en fatigue et,d'autre part, un dispositif de liaison onmidirectionnel; cesdits dispositifs étant conformes à l'invention,
- les figures 7 et 8 montrent respectivement en perspective, un élément de liaison omnidirectionnel et un élément de liaison directionnel selon l'invention.
- La figure 9 montre un ensemble réel testé et la fig.10 les résultats de l'essais.
- Les figures 11 et 12 représentent des radiographies aprés pose.

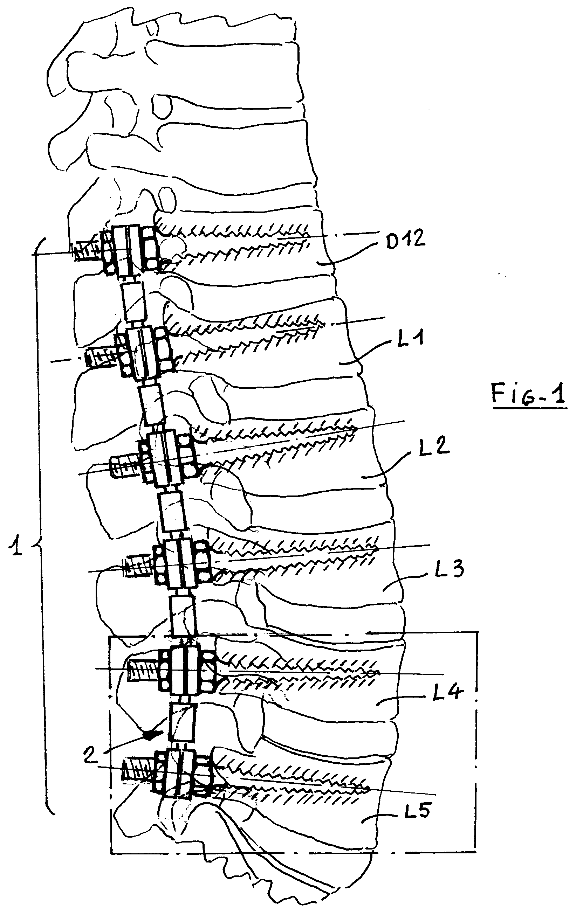

Si l'on se reporte à la figure 1, on voit représenté partiellement à titre d'exemple sur un rachis et entre les vertèbres L5 et D12, des éléments de liaison intervertébraux selon l'invention avec leurs vis pédiculaires, leurs moyens d'ancrage et leurs propres dispositifs de liaison.

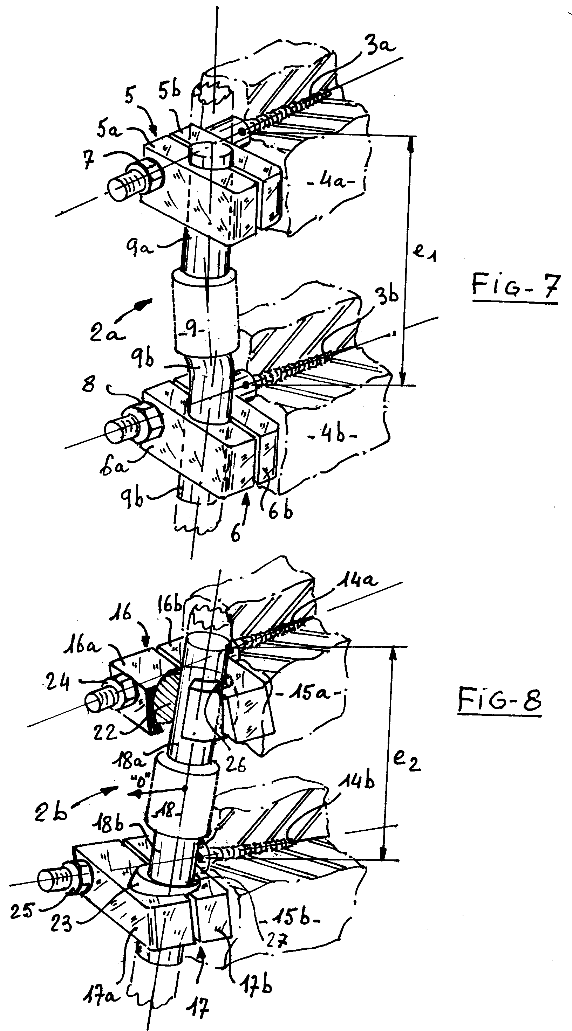

Ces éléments de liaison repérés 2 constituent un ensemble de liaison repéré 1 et peuvent, dans le sens de l'invention, être indiffèremment conformés selon les figures 7 ou 8 comme cela sera explicité dans la suite du texte.

Ils peuvent en outre conformèment à l'usage, être disposés de part et d'autre de l'épine dorsale bien que la plupart des dessins ne représentent que l'un de ces côtés.Comme représenté schèmatiquement sur la figure 2 on montre les charges et contraintes mécaniques que peuvent subir in vivo les vertébres L5-L4 prises comme référence.

Ces vertèbres comportent un disque d'interposition di, composé du nucléus pulposus entouré de l'anulus qui est une substance fibreuse, et les points d'appui sont représentés en p2-p3 par les apophyses articulaires et en p1 par le centre de pression appliqué sur la partie centrale du nucléus pulposus.

A l'intérieur de ces points d'appuis, se trouve situé un point virtuel v d'application de la force instantannée de compression F et pouvant s'inscrire dans un ellipsoïde E normalement contenu dans ce triangle p1-p2-p3,mais pouvant s'en écarter par suite d'une déficience mécanique desdits points d'appuis.

A l'état fondamental normal,le triangle p1-p2-p3 définit les plans d'assiette x-x'/y-y' horizontalement et z-z' verticalement.

Considérant que le rachis peut assumer normalement des débattements tels que:

| zône concernée | Flexion | Extension | Inclinaison latérale. | Rotation |
|---|---|---|---|---|
| -rachis cervical | 60° | 64° | 80° | 165° |
| -rachis dorsal | 35° | 50° | peu étendu | 20° |
| -rachis lombaire | 60° | 45° | Limité | Limité |

on admet qu'un dépassement significatif de ces valeurs moyennes représentée symboliquement par les variations représentées par a et/ou b, et/ou c, sur la figure 2, peut entraîner des déformations importantes relevables d'une intervention éventuelle de pose d'une prothèse.

Se superposent par ailleurs à ces déformations, des contraintes cycliques comme cela a déja été mentionné.

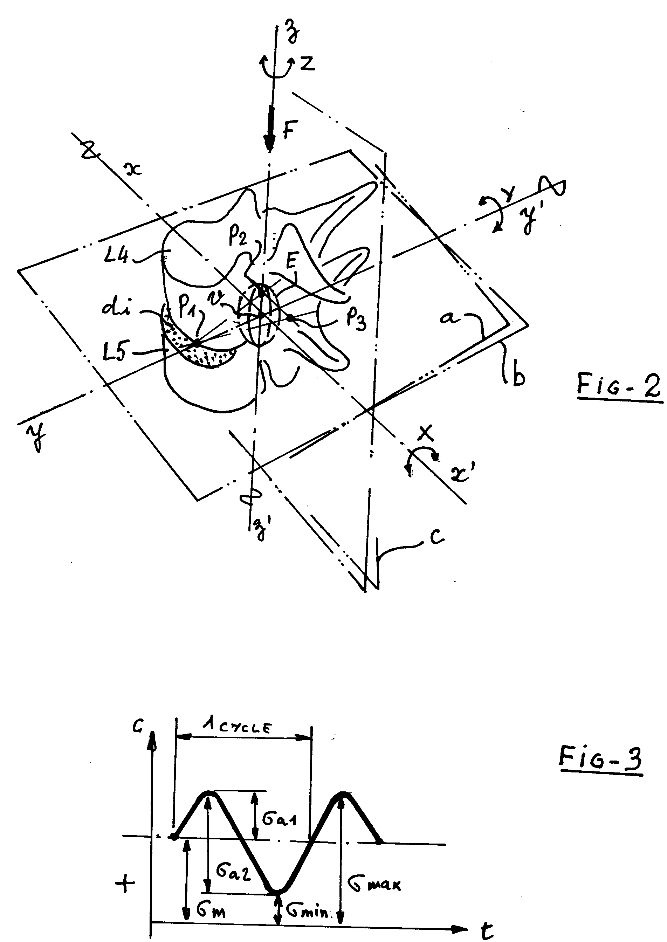

La figure 3 montre à cet effet en référence aux symboles usités en résistance des matériaux, les contraintes pouvant s'exercer selon X-Y-Z autour des axes principaux.

Ces symboles sont notés:
. $\sigma$ max. -contrainte maximale qui est la plus grande valeur algébrique au cours d'un cycle de contrainte (traction ou tension +,compression ou pression -),
. $\sigma$ min. -contrainte minimale qui est la plus petite valeur algébrique au cours d'un cycle de contrainte,
. $\sigma$ m -contrainte moyenne qui est la composante statique de la contrainte et la moyenne algébrique des contraintes $\sigma$ max et $\sigma$ min,
. $\sigma$ $a_{1,2}$ -amplitude de la contrainte qui est la composante alternée de la contrainte, demi différence al-

3

gébrique entre σ max et σ min,

. f -fréquences de cycles (min. sec.)

Résistance à la fatigue (d'aprés ISO R 373- 1964)

. N -endurance ou longévité à la fatigue correspondant au nombre de cycles nécessaires pour provoquer la rupture (généralement exprimée en multiples de $10^6$)

. σ n -résistance à la fatigue pour N cycles ou valeur de la sollicitation pour laquelle le dispositif présenterait une longévité de N cycles.

. σ D -limite de fatigue, déterminée statistiquement et qui peut être illimitée,

. δ - contrainte de cisaillement.

On a reporté sur la figure 2 symboliquement, les contraintes exercées et représentées sur la figure 3.

La partie descriptive donnée à titre de forme de réalisation, va montrer comment l'invention peut être mise en oeuvre à travers la suite du texte.

En se reportant à la figure 1, on rappelle qu'un ensemble prothètique rachidien selon l'invention, repéré 1 dans son ensemble, comporte en nombre convenable des éléments prothétiques de liaison 2 disposés généralement de part et d'autre de l'épine dorsale.

Dans l'exemple, l'ensemble 1 englobe les vertébres L5 à D12.

Chaque élément de liaison 2 peut comporter, selon les besoins de la cause, une conformation soit omnidirectionnelle 2a comme représenté sur la figure 7, soit directionnelle 2b comme représenté sur la figure 8.

Un élément de liaison omnidirectionnel 2a, comprend en premier lieu, deux vis pédiculaires 3a-3b assujetties sur chacune des vertébres 4a (haute) et 4b (basse) et leur partie dépassante filetée reçoit des moyens d'ancrage 5-6 constitués de deux demi-colliers 5a-5b et 6a-6b serrés par des écrous 7 et 8.

L'élément est complété par un dispositif de liaison 9 omnidirectionnel possèdant des queues 9a-9b pouvant être tordues par des outils chirurgicaux classiques au moment de la pose, pour venir s'adapter trés exactement entre les demi colliers des moyens d'ancrage 5 et 6.

l'ensemble ainsi constitué est bloqué par les écrous 7-8 aprés mise en place en tenant compte du réglage et du positionnement intervertébral e1.

Un tel élément 2a est dit omnidirectionnel en ce sens que le dispositif de liaison 9 n'a pas d'orientation angulaire privilégiée comme cela ressort de l'examen de la figure 6.

Sur cette figure 6 on voit que le dispositif de liaison 9 comporte des queues 9a-9b et présente un corps de révolution oblong creux 10 tel que cylindrique, fendu hélicoidalement afin de le rendre élastique axialement et la section 11 présente une allure fermée de préférence rectangulaire tandis qu'aux parties supérieures et inférieures sont fixées lesdites queues 9a-9b et que la partie centrale creuse 12 dudit corps est remplie au repos d'un produit viscoélastique d'amortissement flué en débordement interfente.

Un élément de liaison directionnel 2b comprend également, en premier lieu, deux vis pédiculaires 14a-14b assujetties sur chacune des vertébres 15a (Haute) et 15b (basse) et leur partie dépassante filetée, reçoit des moyens d'ancrage 16-17 constitués de demi-colliers 16a-16b/17a-17b serrés par des écrous 24-25.

Ces demi-colliers ont une empreinte sphérique afin d'enserrer des noix cylindro-sphériques 22-23 avec fentes 26-27 recevant les queues 18a-18b du dispositif de liaison 18 et les écrous 24-25 assurent par serrage et aprés réglage de l'espace intervertébral e2 et le positionnement angulaire correct selon "0", le blocage de l'ensemble.

le corps 20 du dispositif de liaison 18 qui peut être cylindrique, comporte des fentes transversales dissymétriques 21 garnies au repos d'un produit viscoélastique 22 flué en débordement interfentes.

Un tel élément 2b est dit directionnel en ce sens que le nombre, la largeur, la profondeur et les orientations angulaires des fentes 21 permet par un positionnement convenable "0", de corriger élastiquement certains défauts tels que scolioses par exemple.

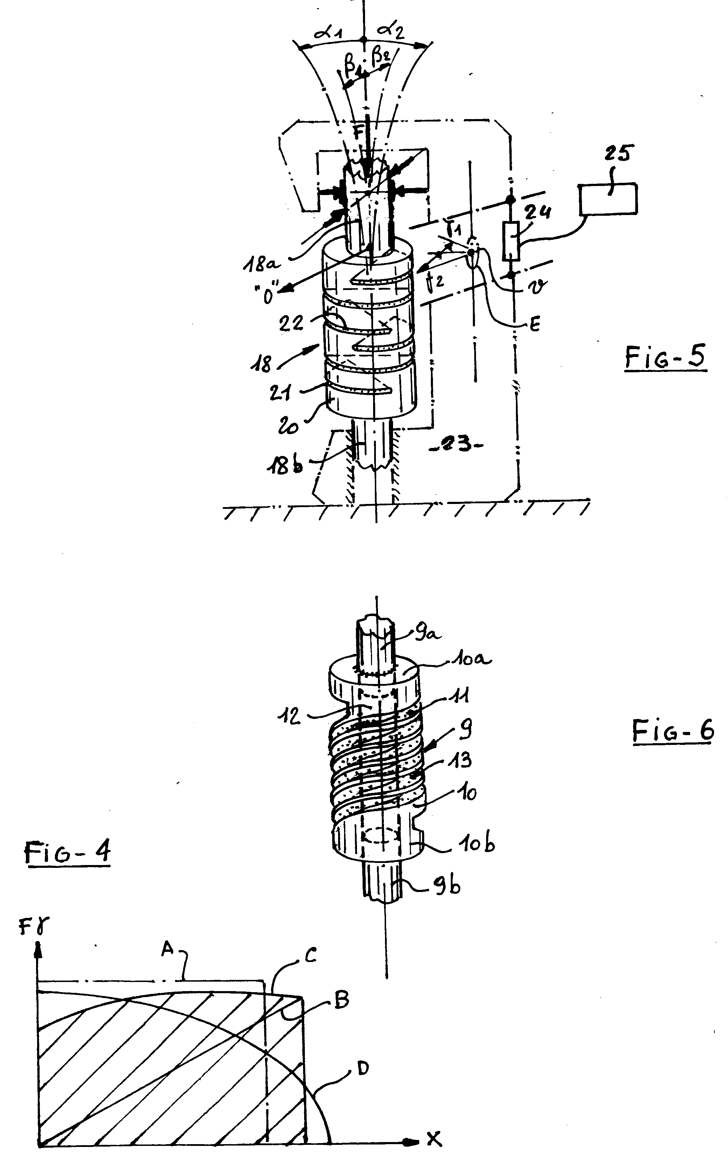

La figure 5 montre comment les dispositifs de liaison 2a ou 2b peuvent être testés en fatigue aprés encastrement de leurs queues 9a-9b ou 18a-18b dans les mors d'un banc d'essais repéré schèmatiquement 23 sur cette figure et dont les éléments mécaniques sont aisèment concevables.

Un tel banc permet d'afficher pratiquement tous les paramètres utiles à la définition des dispositifs de liaison selon l'invention.

Ainsi on peut définir à titre d'exemple :

- la force d'application axiale F estimée à 100 daN,

- la flexion latérale sens x-x' : α 1=4°- α 2=4°

- la flexion latérale sens y-y' : β 1=4°- β 2=4°

- la rotation autour de l'axe z-z' : γ 1=1°- γ 2=1°

pour un nombre de cycles dépassant $10^6$.

les essais sont conduits en contrôlant le positionnement du point v par rapport à l'ellipsoide E ainsi que l'orientation "0" et toutes les informations sont recueillies par un capteur 24 et traitées par une chaîne de mesures 25.

Les demandeurs ont conduit sur banc d'essais adapté, des essais permettant de définir un ensemble rachidien prothétique biomécanique proche de celui existant in-vivo.

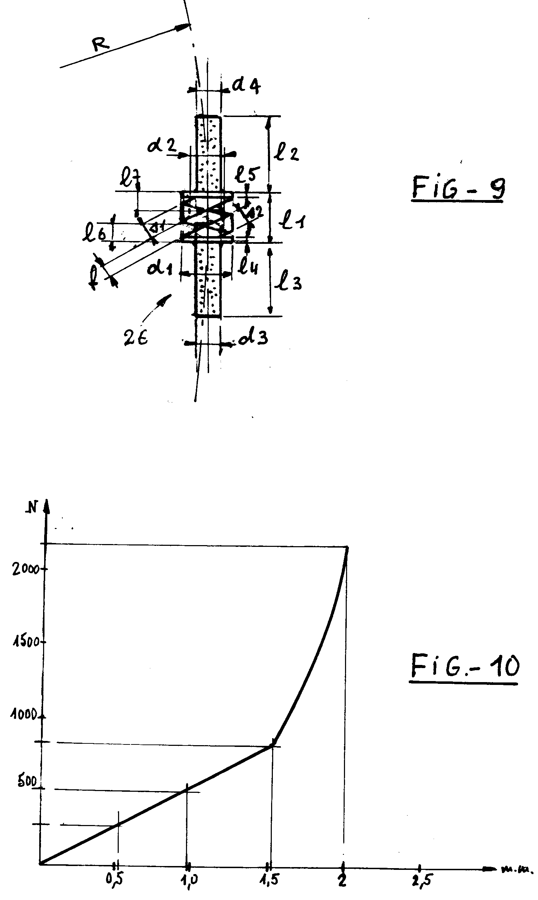

La figure 9 montre les caractéristiques dimensionnelles du disposifif mis en oeuvre et la figure 10 les résultats d'essais obtenus sous forme d'un enregistrement graphique.

Selon cette figure 9, ledit dispositif prothétique présente les caractéristiques dimensionnelles suivantes (en mm.): d1 (ext)= 13/ d2 (int)= 7/ d3=d4= 6/ℓ1 =12/ℓ2= 3= 18/ℓ 4=ℓ5= 1,5/ s1= 4/ s2= 2,5/ f= 2,5 /ℓ 6=ℓ7= 3,8 et les disques d'extrémités d'ep 1,5 ont été soudés par faisceaux d'électrons sur les queues qui par ailleurs présentent des pointes de diamant en surface pour empêcher la rotation axiale aprés fixation.

Un précambrage de R=220 mm. peut être pratiqué pour faciliter la pose.

La figure 10 montre la courbe résultante de l'essais pratiqué sur l'ensemble dimensionné ci-dessus et constitué d'un acier 5832/3 ISO biocompatible poli miroir.

Sur cette figure, on note les points caractéristiques suivants:

```
    - pour 1 mm. de déformation - charge appliquée: 500 N,

    - "    1,5 mm.       "         -  "        "    : env.  800 N,

    - "    2 mm.         "         -  "        "    : env. 2 200 N.
```

Ces points caractéristiques sont trés proches de ceux résultant du comportement biomécanique humain selon les conceptions généralement admises.

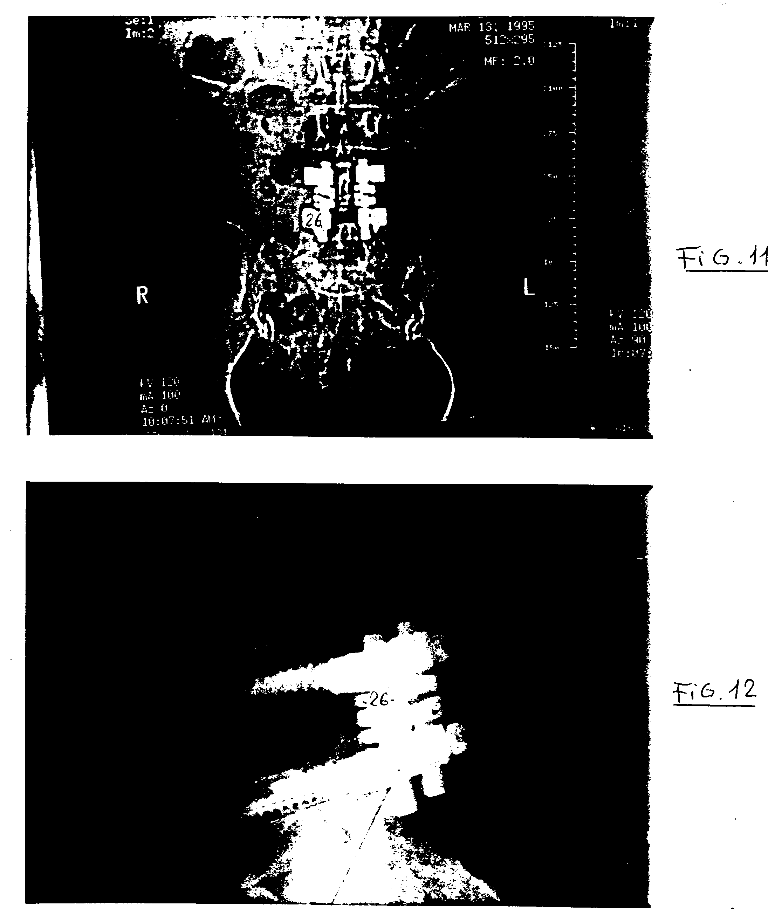

Les figures 11 et 12 représentent, à titre d'exemple de réalisation, deux radiographies de face et de profil de l'implantation récente effectuée par l'un des demandeurs qui est le Docteur ELBERG, d'un ensemble prothétique rachidien conforme à l'invention (26) sur un patient agé de 60 ans.


## Revendications

-1-/ Ensemble prothétique rachidien 1 caractérisé en ce qu'il comporte en nombre convenable, des éléments prothétiques de liaison intervertébraux élastiques avec amortissement (2) présentant selon les besoins une conformation, soit omnidirectionnelle (2a) comprenant au moins deux vis pédiculaires (3a-3b) assujetties l'une sur la vertébre dite haute (4a)l'autre sur la vertébre dite basse (4b) et recevant des moyens d'ancrage (5-6) réunis par un dispositif de liaison omnidirectionnel à queues (9),soit directionnelle (2b) comprenant au moins deux vis pédiculaires de même genre (14a-14b) assujetties l'une sur la vertébre dite haute (15a) l'autre sur la vertébre dite basse (15b) et recevant des moyens d'ancrage (17-18) réunis par un dispositif de liaison directionnel à queues (18), et chaque élément (2) est paramétré et normalisé avant pose par référence à des essais préalables sur banc de mesures.

-2-/Ensemble prothètique rachidien selon la revendication 1 caractérisé en ce que l'élément prothétique de liaison omnidirectionnelle (2a) comporte un dispositif de liaison (9) présentant un corps de révolution oblong creux (10) tel que cylindrique, fendu hélicoidalement pour le rendre élastique axialement et la section spiralée (11) présente un allure polygonale de préférence rectangulaire, tandis qu'aux parties supérieure (10a) et inférieure (10b) dudit corps sont assujetties des queues haute (9a) et basse (9b) destinées à être fixées aprés cambrage convenable in situ sur des moyens d'ancrage (5-6) alors que la partie centrale creuse (12) dudit corps (10) est remplie au repos d'un produit viscoélastique d'amortissement (13) flué en débordement interfente.

-3-/ Ensemble prothétique rachidien selon la revendication 1 caractérisé en ce que l'élément prothétique de liaison directionnelle (2b) comporte un dispositif de liaison (18) présentant un corps de révolution tel que cylindrique possèdant, d'une part, en partie centrale des fentes transversales dissymétriques (21) en nombre de largeur de profondeur et d'orientations convenables afin de le rendre élastique directionnellement dans le sens axial après orientation convenable selon "0" et d'autre part,en ses parties extrémales, des queues cylindriques haute (18a) et basse (18b) liées à des moyens d'ancrage (16-17) tandis que les fentes (21) sont remplies au repos d'un produit viscoélastique d'amortissement (21) flué en débordement interfente.

-4-/ Ensemble prothétique rachidien selon la revendication 2 caractérisé en ce que les moyens d'ancrage (5-6)comprennent sur la partie dépassante filetée des vis pédiculaires (3a-3b) des demi-colliers à empreintes circulaires (5a-5b) et (6a-6b) enserrant les queues cylindriques haute (9a) et basse (9b) garantissant aprés serrage des écrous (7-8) l'immobilisation totale de l'ensemble de l'élément de liaison (20).

-5-/ Ensemble prothétique rachidien selon la revendication 3 caractérisé en ce que les moyens d'ancrage (16-17) comprennent sur la partie dépassante filetée des vis pédiculaires (14a-14b) des demi-colliers à empreinte sphérique (16a-16b/17a-17b) enserrant des noix cylindrico-sphériques fendues (22-23) recevant les queues cylindriques haute (18a) et basse (18b) garantissant, aprés orientation radiale convenable selon "0" et réglage selon e2, par le serrage des écrous (24-25), une immobilisation totale de l'ensemble de l'élément de liaison (2b).

-6-/ Ensemble prothétique rachidien selon l'une quelconque des revendications 1 à à 5 caractérisé en ce que les queues (9a-9b/18a-18b) peuvent être communes à deux éléments de liaison adjacents.

-7-/ Ensemble prothétique rachidien selon l'une quelconque des revendications 1 à 5 caractérisé en ce que les moyens d'ancrage (5-6) ou (16-17) sont agencés en largeur pour pouvoir recevoir deux queues.

-8-/ Ensemble prothètique rachidien selon l'une quelconque des revendications 1 à 6 caractérisé en ce que son paramètrage consiste:

- à déterminer par tous moyens appropriés (radiographie,imagerie RM,scanner....) les défauts à corriger en rapport avec l'anomalie constatée,
- à analyser ces défauts pour en dégager les paramètres correctifs,
- à modéliser la prothèse en fonction des paramètres correctifs relevés,
- à soumettre la prothèse modélisée aux essais sur banc de fatigue,
- à normaliser le modèle de prothèse testé aux essais afin d'être à même de reconstituer, en fonction de chaque cas considéré, le type de prothèse répondant exactement à l'application chirurgicale envisagée,
- à implanter ladite prothèse.

-9-/ Ensemble prothétique selon l'une quelconque des revendications 1 à 8 caractérisé en ce que le banc de mesures en fatigue ((23) comporte sur un bâti:

- un moyen de fixation des queues (9a-9b/18a-18b),
- un dispositif d'application de contraintes variables sous charge F dans des limites de déplacement du point vurtuel (v) à l'intérieur de l'éllipsoide (E) qui est représentative des valeurs limites admissibles ($\alpha 1$-$\alpha 2/\beta 1$-$\beta 2/\gamma 1$-$\gamma 2$),
- un capteur tridimensionnel (24),
- une chaîne de mesures (25).

-10-/ Ensemble prothétique selon l'une quelconque des revendications 1-2-4-6-7-8-9- caractérisé en ce qu'il se présente sous la forme omnidirectionnelle (26) avec comme dimensions (en mm.): d1 (ext)=13/ d2 (int)=7/ d3=d4=6/ $\ell 1$=12/ $\ell 2$=$\ell 3$=18 $\ell 4$=$\ell 5$=1,5/ s1=4/ s2=2,5/ f=2,5/ $\ell 6$=$\ell 7$=3,8/ et les disques d'extrémités sont soudés par faisceaux d'électrons sur les queues qui par ailleurs comportent des pointes de diamant en surface; ledit ensemble présentant aux essais des points caractéristiques trés proches de ceux du comportement biomécanique humain.

D12

L1

Fi6-1

L2

1

L3

2

L4

L5

FIG-2

FIG-3

FiG-5

FiG-6

FiG-4

9

FIG-7

FIG-8

FIG - 9

FIG.- 10

FiG. 11

FiG. 12